# EUROPEAN PATENT APPLICATION

(11) **EP 4 306 109 A1**
(43) Date of publication of application: **17.01.2024**
(21) Application number: 22315142.4
(22) Date of filing: 11.07.2022
(51) Int. Cl.: A61K 31/428, A61P 31/04

(54) **NEW ANTIBIOTIC COMPOUNDS**

(71) Applicant: Université de la Côte d'Azur, 06100 Nice (FR); Centre National de la Recherche Scientifique, 75016 Paris (FR); Institut National de la Santé et de la Recherche Médicale, 75013 Paris (FR)
(72) Inventor: MUNRO, Patrick, 06100 Nice (FR); PIRES GONCALVES, Leticia, 06100 Nice (FR); BENHIDA, Rachid, 06000 Nice (FR); RONCO, Cyril, 06100 Nice (FR); BOYER, Laurent, 06200 Nice (FR)
(74) Representative: LLR

(57) **Abstract**

Compound with the following formula I: 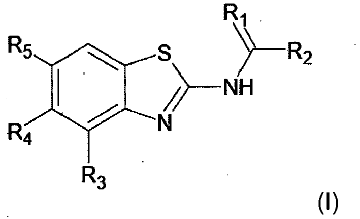 wherein
- R1 represents O or NH,
- R2 represents a linear, branched or cyclic alkyl comprising from 1 to 8 carbon atoms, possibly interrupted by a group selected from -O-, -S-, -(C=O)-, NH and possibly being substituted by one or more halogens;
an alkylene comprising from 1 to 8 carbon atoms and possibly being substituted by one or more halogens,
a phenyl or an arylene,

- R3, R4 and R5 represent independently -H, a halogen, a linear or branched alkyl comprising from 1 to 8 carbon atoms, possibly being substituted by one or more halogens, CN, -NO₂, OX₁ with X₁ being selected from -H, linear or branched alkyl comprising from 1 to 8 carbon atoms, possibly being substituted by one or more halogens, COOR6, OCOR6, SO2NR6R7, NR6R7, NR6COR7, or OR6, with R6 and R7 being identical or different and R6 and R7 representing H or an alkyl comprising from 1 to 8 carbon atoms,
and its use as a medicament.

## Description

The present invention relates to new compounds and to their use as medicament, in particular for use as antibiotic. The invention is also relating to compounds, such as acetamide and acetimidamide derivatives for their use in the treatments of infectious diseases associated with bacteria, and to pharmaceutical compositions containing said compounds which are useful in said treatments and applications of the compounds.

Antibiotics are one of the most important discoveries of medicine to deal with infectious diseases. However, the emergence of multidrug-resistant pathogens towards conventional antibiotics has urged the need of novel antimicrobial agents. This became one of the biggest threats to the healthcare system worldwide and the failure to address this problem could result in 10 million deaths by 2050. Indeed, because of the high selection pressure and of the mis-/overuse of antibiotics, all of them are currently facing resistance issues.

Antibiotic resistance in bacteria may result either from mutations or from the acquisition of resistance genes conferring resistance to one or more antibiotics. Some pathogens are particularly resistant according to the World Health Organization (WHO): Staphylococcus aureus strains that are resistant to a wide range of antibiotics including Methicillin (MRSA) (derived from penicillin beta-lactamic antibiotics) and are thus responsible for severe infections.

Currently, three main mechanisms of resistance are known: (i) enzymatic inhibition of the antibiotic by the bacterium; (ii) decrease in the amount of antibiotics reaching the target, resulting either in a decrease in membrane permeability, or in an expulsion of the antibiotic outside of the bacterium, or destruction; (iii) a modification of the target of the antibiotic

There is therefore a need for new classes of compounds having antibiotics activity, which could overcome the problem of bacterial resistance to existing molecules.

There is also a need to have new antimicrobial compounds which are well-tolerated when administered to an individual, in particular to a mammal and with low cell-toxicity. There is also a need for anti-microbial compounds active on various species of micro-organisms, and possibly less prone to development of resistance.

US 3991210 is disclosing acetamidine derivatives which can be used as antiseptic, and their use in a method for combating microorganisms associated with infections of the urinary tract in mammals.

GB 1482397 is disclosing pharmaceutical compositions containing trichloroacetamidine derivatives, having cardiac stimulant effects.

Kalinin et al (Heterocycles, 2012, 85, 2515-2522) is disclosing synthesis of heteroaryl-trichloroacetamidine, without studying their activity.

Grivas et al (Can. J. Chem. 1961, 39, 761-764) is studying the reaction between halogen susbtituted nitriles and amines.

Hough et al (J. Heterocyclic Chem., 1984, 21(5), 1377-1380) is describing reactions of alkali metal derivative of heterocyclic amines, and is presenting 3 heteroaryl-trichloroacetamidines.

The inventors have now demonstrated that a new class of acetamidine derivatives exhibits a marked antimicrobial activity.

The present invention is therefore directed to compounds of formula (I) 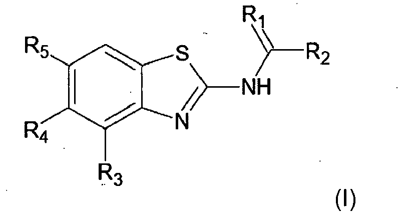 wherein
- R1 represents O or NH,
- R2 represents a linear, branched or cyclic alkyl comprising from 1 to 8 carbon atoms, possibly interrupted by a group selected from -O-, -S-, -(C=O)-, NH and possibly being substituted by one or more halogens;
   an alkylene comprising from 1 to 8 carbon atoms and possibly being substituted by one or more halogens,
   a phenyl or an arylene,
- R3, R4 and R5 represent independently -H, a halogen, a linear or branched alkyl comprising from 1 to 8 carbon atoms, possibly being substituted by one or more halogens, CN, -NO₂, OX₁ with X₁ being selected from -H, linear or branched alkyl comprising from 1 to 8 carbon atoms, possibly being substituted by one or more halogens, COOR6, OCOR6, SO2NR6R7, NR6R7, NR6COR7, or OR6, with R6 and R7 being identical or different and R6 and R7 representing H or an alkyl comprising from 1 to 8 carbon atoms,

as well as the salts or organic complex thereof,
for use as a medicament.

Advantageously, in formula (I), at least one from R3, R4 or R5 represents -H.

The present invention is also directed to compounds of formula (I) 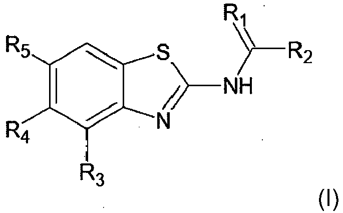 wherein
- R1 represents O or NH,
- R2 represents a linear, branched or cyclic alkyl comprising from 1 to 8 carbon atoms, possibly interrupted by a group selected from -O-, -S-, -(C=O)-, NH and possibly being substituted by one or more halogens;
   or R2 represents an alkylene comprising from 1 to 8 carbon atoms and possibly being substituted by one or more halogens,
   or R2 represents a phenyl or an arylene,
- R3, R4 and R5 represent independently -H, a halogen, a linear or branched alkyl comprising from 1 to 8 carbon atoms, possibly being substituted by one or more halogens, CN, -NO₂, OX₁ with X₁ being selected from -H, linear or branched alkyl comprising from 1 to 8 carbon atoms, possibly being substituted by one or more halogens, COOR6, OCOR6, SO2NR6R7, NR6R7, NR6COR7, or OR6, with R6 and R7 being identical or different and R6 and R7 representing H or an alkyl comprising from 1 to 8 carbon atoms,

as well as the salts or organometallic complex thereof,
for use as an antibiotic.

Advantageously, in formula (I), at least one from R3, R4 or R5 represents -H.

Compounds of formula (I), the salts or organometallic complex thereof are also a subject matter of the invention, with the exception of compounds having the following formula

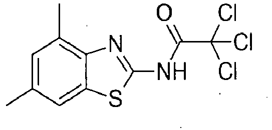

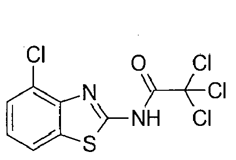

Compounds of formula (I) according to the invention, may in particular have the definitions below:
- R1 represents O or NH,
- R2 represents a linear, branched or cyclic alkyl comprising from 1 to 8 carbon atoms, possibly interrupted by a group selected from -O-, -S-, -(C=O)-, NH and possibly being substituted by one or more halogens;
   an alkylene comprising from 1 to 8 carbon atoms and possibly being substituted by one or more halogens,
   a phenyl or an arylene,
- R3, R4 and R5 represent independently -H, a halogen, a linear or branched alkyl comprising from 1 to 8 carbon atoms, possibly being substituted by one or more halogens, -NO₂, or OX₁ with X₁ being selected from -H, linear or branched alkyl comprising from 1 to 8 carbon atoms, possibly being substituted by one or more halogens.,
Advantageously, in formula (I), at least one from R3, R4 or R5 represents -H.

Advantageously, R2 represents a linear or branched alkyl comprising 1 to 5 carbon atoms, substituted by at least one halogen. Preferred halogens are selected from Cl, F, and Br. According to the invention, R2 may in particular represent a linear or branched alkyl comprising 1 to 5 carbon atoms, substituted by 3 halogens.

R2 may therefore be selected from'alkyl in C1, C2, C3, C4 and C5, in particular in C1 or C2. R2 can in particular represent a C1 alkyl, substituted by 1, 2 or 3 halogens. In this last case, R2 represents C(R'1)₃, wherein R'1 represents a halogen. Preferably, R'1 represents Cl, F or Br, in particular R'1 represents Cl. Advantageously, in compounds according to the invention, R2 is therefore representing C(Cl)₃.

In formula (I) as defined above, at least one of R3 or R5 may in particular be selected from halogen and a linear or branched alkyl comprising from 1 to 6 carbon atoms, possibly being substituted by one or more halogens, or a halogen, and R4 represents H. R3 and R5 may be identical or different; as mentioned above, one of R3 or R5 may also represent H.

Advantageously, R3 represents a halogen or a linear or branched alkyl comprising 1 to 3 carbon atoms.

According to one embodiment of the invention, at least one of R3 or R5, identical or different, represents a halogen, in particular a halogen selected from Cl, F and Br, the other groups in formula (I) being as defined elsewhere in the application. Preferably, R4 represents H. In particular, both R3 and R5 can represent a halogen, or at least one of R3 or R5 represents H.

Such compounds exhibit advantageous antimicrobial activities.

In another embodiment of the invention, at least one of R3 or R5, identical or different, represents a linear or branched alkyl comprising from 1 to 6 carbon atoms, in particular from 1 to 3 carbon atoms, the other groups in formula (I) being as defined elsewhere in the application. Preferably, R4 represents H. In particular, at least one of R3 or R5 represents a methyl; accordingly, both R3 and R5 can represent a methyl, or at least one of R3 or R5 represents H.

Compounds wherein in formula (I) as defined above, R1 represents NH, are of particular interest.

Compounds according to the invention are in particular the following ones:

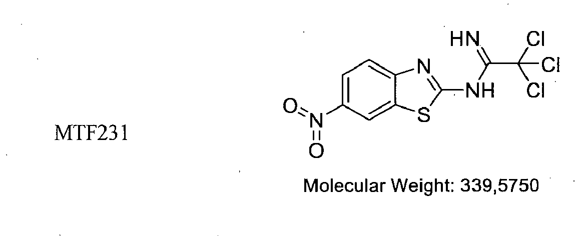

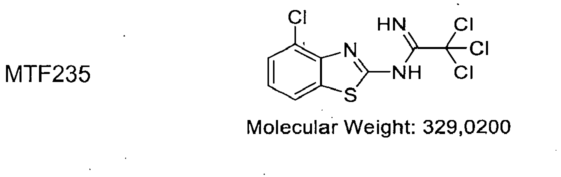

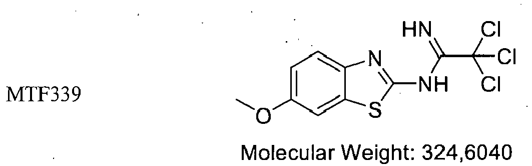

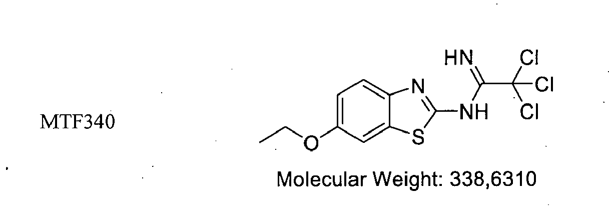

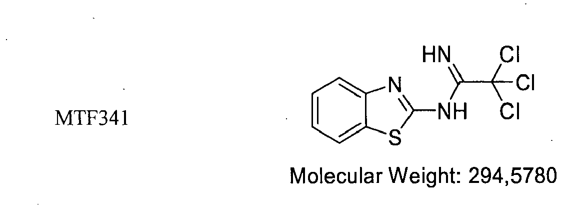

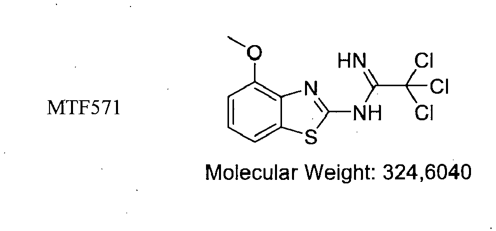

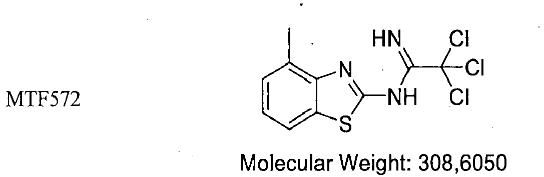

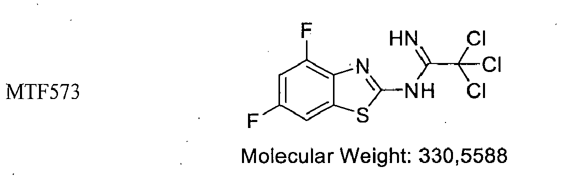

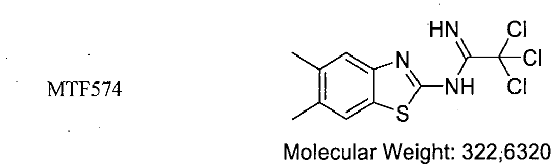

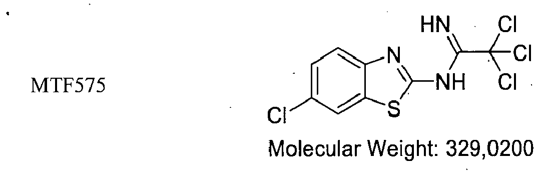

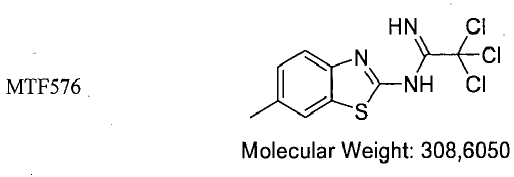

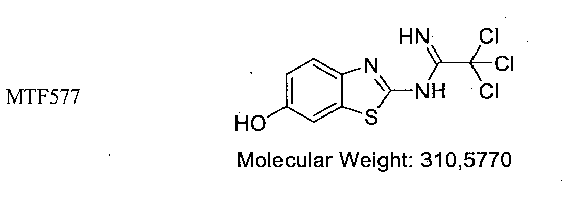

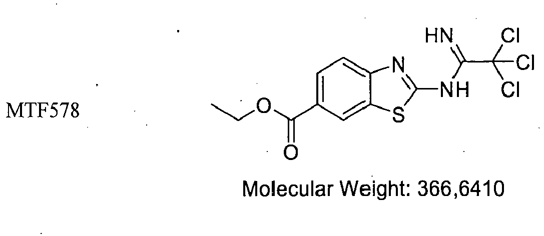

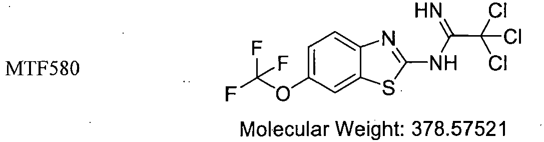

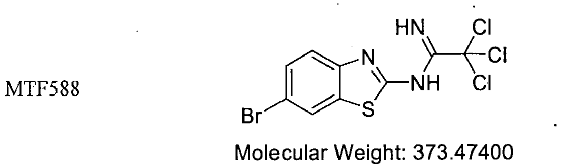

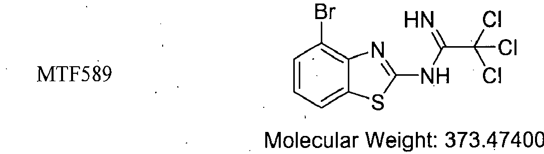

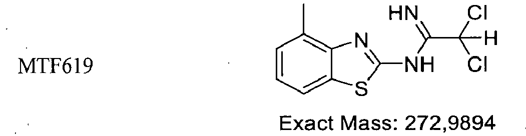

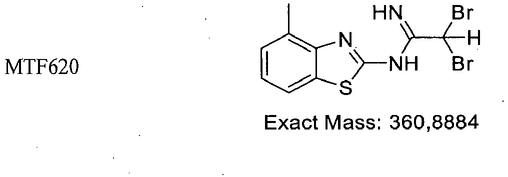

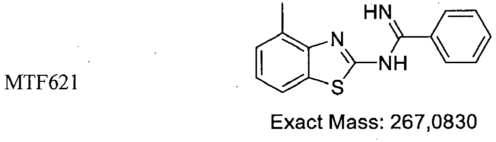

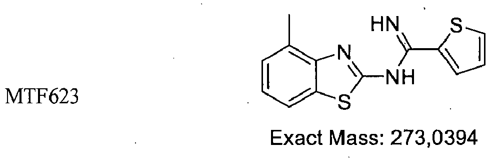

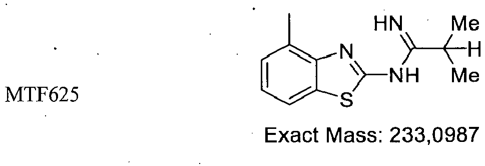

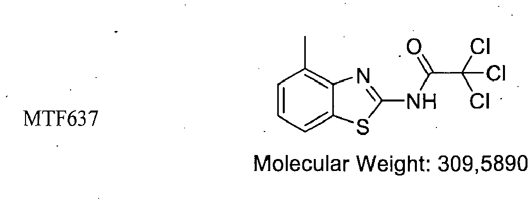

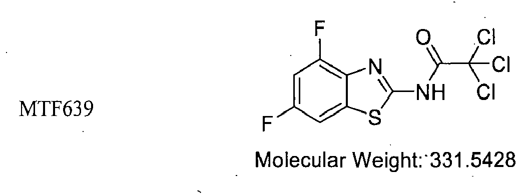

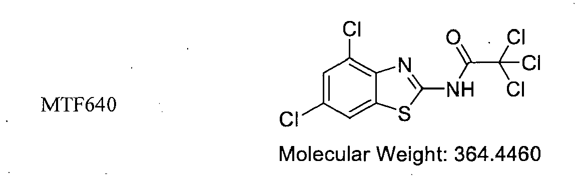

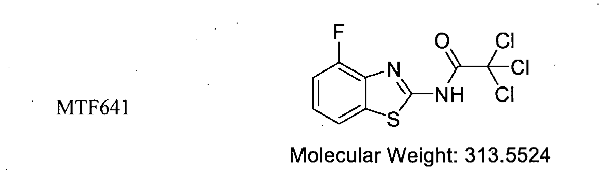

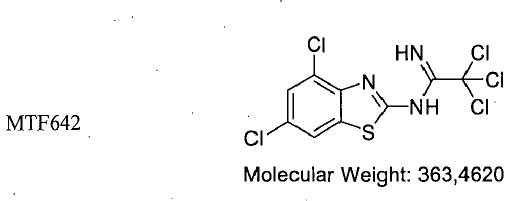

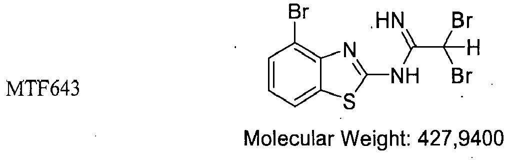

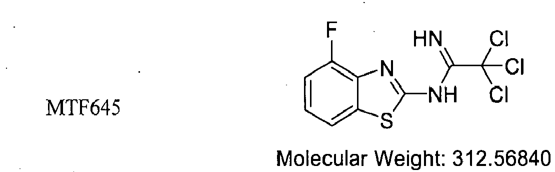

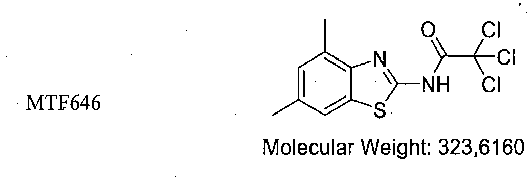

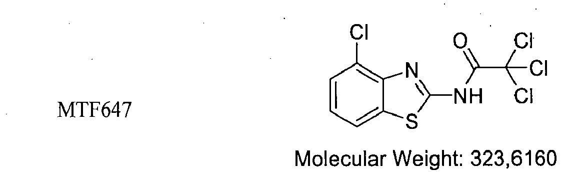

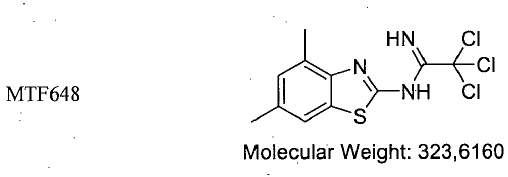

Compounds of formula (I) according to the present invention have in particular one of the following formulas: 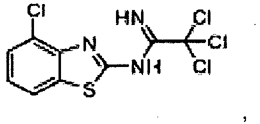 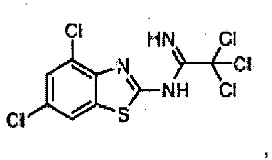 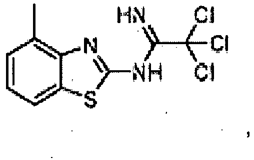 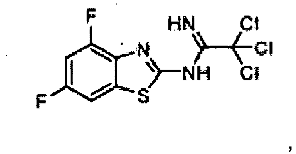 and

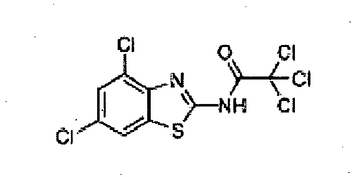

Compounds of formula (I) wherein R1 represents NH can be prepared for instance by adding the corresponding 2-aminobenzothiazole to a nitrile derivative of formula NCR2, in a suitable solvent. Then, the desired product is recovered by filtration, liquid-liquid extraction or by evaporation of the volatiles under reduced pressure. The crude product can be recrystallized in a suitable solvent mixture to obtain a pure product.

In particular, when R2 represents CCl3, the synthesis reaction may be as follows:

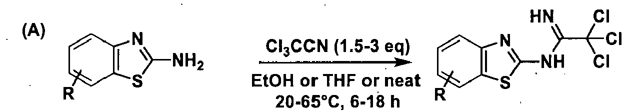

Compounds of formula (I) wherein R1 represents O or R2 represents a group different than CCl3 can be prepared as follows:

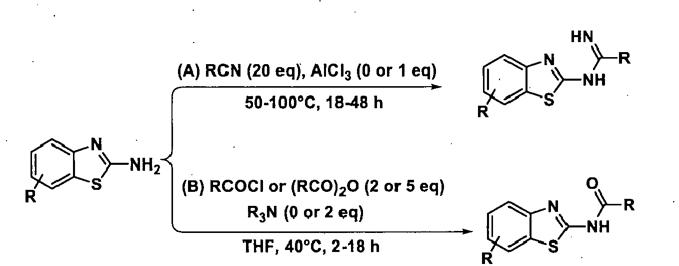

Compounds of formula (I) as defined throughout the present specification have been found as useful as medicament.

As mentioned before, the expression "compounds of formula (I)" is intended to encompass also their pharmaceutically acceptable salts and derivatives and in particular complex of compounds of formula (I) with metals and/or metallic ions. These organo-metallic compounds may have improved properties. Metals may be selected from alkali metals, alkaline earth metals, transition metals, post-transition metals, lanthanides and actinides.

Compounds of formula (I) may be in particular be in the form of a complex with at least a metal selected from transition metals, for instance a metal selected from Cu, Zn and Mo.

It has now been demonstrated that compounds of formula (I) as defined above exhibit good ant-bacterial activity, while being well tolerated as having low cytotoxicity for healthy cells. They are stable and can be formulated in compositions for long term use.

The invention is therefore directed to compounds of formula (I) as defined here-above, their salts, derivatives or metallic complex, for their use as antibiotics.

Antibiotic is designating an anti-microbial compound or composition, active against bacteria. Antibiotics are able to fight bacterial development, either by killing bacteria or by lowering, reducing or stopping their growth. They can therefore act as bactericide or as bacteriostatic. In some case, a same substance can be a bactericide substance or a bacteriostatic substance, depending on the concentration used.

The present invention is in particular relating to the use of compounds of formula (I) as defined above, as bactericide.

The compounds of formula (I) are in particular used in the treatment of bacterial infections, i.e. in preventing, reducing or stopping bacterial infections in a subject in need thereof.

Said subject is advantageously a mammal, and in particular a human being. The term "patient" can also be used in the present specification, to refer to the subject, in particular human being, treated with the compound of formula (I) according to the invention. The term individual may also be used.

The invention is therefore relating to a compound of formula (I) for its use in the treatment of an infectious disease associated with bacteria, in particular an infectious disease associated with at least a Gram positive (Gram +) or a Gram negative (Gram -) bacteria.

Compounds of formula (I) may be given as preventive measure, for instance in at-risk population, or in surgical procedures to help to prevent infection of incision or in dental procedures.

Compounds of formula (I) are used according to the invention in preventing or reducing the development of a bacterial infection. By "reducing" is intended the use in limiting partly or completely the development of an infection; accordingly, in some embodiments, a compound of formula (I) will stop the development of infection at an early stage. Reducing is also comprising delaying or slowering the development of the infection.

By preventing is intended the use before the onset of the infection, so that the bacterial infection will not appear.

Except if otherwise stated, the expression "a compound of formula (I)" is intended to mean "at least one compound of formula (I)". Accordingly, it encompasses either the use of only one compound of formula (I) or of mixtures of two or more compounds of formula (I).

As indicated above, compounds of formula (I) according to the invention are useful in the treatment of infections associated or induced by bacteria. Compounds of formula (I) as defined in the present application are in particular used for the treatment of infectious diseases associated with Gram-positive bacteria.

Gram-positive bacteria can be responsible for various pathologies; Gram-positive bacteria can also be present on some location of the organism as resident flora, without being responsible of pathology. Infection may occur either with specific pathogenic bacteria, or when the balance between the bacteria present at a location is disturbed.

Compound of formula (I) can be used in infectious'disease or pathologies associated with Gram-positive bacteria selected from gram-positive cocci and gram-positive bacilli (rods).

Gram-positive bacteria comprise the genera *Staphylococcus* and *Streptococcus.* Gram-positive bacilli comprise the genera *Bacillus* and *Clostridia* (spore-forming rods), *Listeria* and *Corynebacterium.* Also, *Rathybacter, Leifsonia,* and *Clavibacter* are three gram-positive genera that cause plant disease.

Compounds of formula (I) according to the invention can be used in particular for the treatment of gram-positive bacterial infections or diseases, wherein the bacteria is selected from *Staphylococcus aureus, Staphylococcus epidermidis, Streptococcus pneumoniae, Streptococcus pyogenes, Streptococcus agalactiae, Clostridia tetani, Clostridia botulinum, Clostridia perfringens, Clostridia difficile, Bacillus anthracis, Bacillus cereus, Corynebacterium diphtheria* and *Listeria monocytogenes.*

Compounds of formula (I) are particularly adapted for the treatment of bacterial disease associated with *Staphylococcus.*

Such diseases are including inflammatory diseases, including skin infections, pneumonia, endocarditis, septic arthritis, osteomyelitis, and abscesses. Compound of formula (I) are also useful for the treatment of genital or urinary tract infections, otitis media, pneumonia, sinusitis, meningitis, endocarditis, impetigo, erysipelas, glomerulonephritis and rheumatic fever, biliary tract infections or diphteria.

Compound of formula (I) may for instance be used in the treatment of skin disease, such as impetigo.

The invention is also relating to pharmaceutical compositions containing at least a compound of formula (I) and at least a physiologically acceptable medium in particular pharmaceutical acceptable carriers. It is understood that all the use and applications of compounds of formula (I) may be in a composition containing said compound or derivative thereof.

As mentioned above, a compound of formula (I) may be used in a composition containing said inhibitor, in particular a pharmaceutical composition. The compound of formula (I) is administered to a subject in need thereof through routes adapted by the man skilled in the art.

Various delivery systems are known and can be used to administer a compound of formula (I) of the present invention, including tablets, capsules, injectable solutions, encapsulation in liposomes, microparticles, microcapsules, etc. Methods of administration include, but are not limited to, dermal, intradermal, intramuscular, intraperitoneal, intralesional, intravenous, subcutaneous, intranasal, pulmonary, epidural, ocular, and oral routes. A compound of formula (I) of the present invention, or a pharmaceutical composition thereof, may be administered by any convenient or other appropriate route, for example, by infusion or bolus injection, by adsorption through epithelial or mucocutaneous linings (e.g. oral, mucosa, rectal and intestinal mucosa, etc). Administration can be systemic or local. Parenteral administration may be directed to a given tissue of the patient, such as by catheterization. As will be appreciated by those of ordinary skill in the art, in embodiments where a compound of formula (I) is administered along with an additional therapeutic agent, the compound of formula (I) and the therapeutic agent may be administered by the same route (e.g. , orally) or by different routes (e.g., orally and intravenously).

A compound of formula (I) can be administered in a composition comprising one or more pharmaceutically acceptable diluents or carriers.

A "pharmaceutical composition" is defined herein as comprising an effective amount of at least one compound of formula (I) of the invention, or a physiologically tolerable salt or derivative thereof, and at least one pharmaceutically acceptable carrier or excipient.

The term "pharmaceutically acceptable carrier or excipient" refers to a carrier medium which does not interfere with the effectiveness of the biological and/or therapeutic activity of the active ingredient(s) and which is not excessively toxic to the host at the concentration at which it is administered. This term includes solvents, dispersion media, coatings, antibacterial and antifungal agents, isotonic agents, and adsorption delaying agents, and the like. The use of such media and agents for pharmaceutically active substances is well known in the art).

The invention will be better understood with the following examples. The examples are referring to figures

### Description of figures

Fig. 1 is representing the results of bacteriostatic and bactericidal activities obtained on two Staphylococcus aureus strains, RN4220 and S25.
Fig. 2 is representing the results of cytotoxicity evaluation through extracellular lactate dehydrogenase (LDH) quantification upon incubation of the compounds with HEK 293T cells at the same range of concentration as MIC and MBC. LDH: positive control, DMEM: negative control. N = 3.

### Example 1 : Preparation of compounds of formula (I)

### Material and methods

All chemicals were purchased from Sigma-Aldrich, Alfa Aesar, Apollo Scientific and Carlo Erba and used without further purification. Thin layer chromatography (TLC) was performed on precoated Merck 60 GF254 silica gel plates and revealed first by visualization under UV light (254 nm and 360 nm). 1H, 13C and 19F NMR spectra were recorded on a Bruker Advance 400 MHz or a Bruker Advance 200 MHz spectrometer. Mass spectra (ESI-MS) were recorded on an Agilent iQ (Agilent, Santa Clara) single quadrupole mass spectrometer, fitted with an Electrospray source and hyphenated with an Agilent 1200 HPLC system. HRMS spectra were recorded on a ThermoFisher Q-Exactive Focus mass spectrometer (ESI-MS) at the resolution of 70 000 at m/z 200. The mass spectrometer is hyphenated with a ThermoFisher Vanquish UHPLC system. The purity of compounds was further assayed by HPLC analysis on a JASCO PU-2089/AS4050 apparatus with a Supelco analytical column Ascentis Express C18, 100 mm × 46 mm 5 µM, employing the flowing two methods:
Method 1: 5% B for 3 min, 5% B to 95% B over 12 min, 95% B for 5 min then from 95% B to 5% B over 2 min, 5% B for 3 min (total time: 25 min). Flow: 1 mL/min. Solvent A: water with 0.1% formic acid. Solvent B: acetonitrile with 0.1% formic acid.
Method 2: 30% B for 2 min, 30% B to 95% B over 6 min, 95% B for 12 min then from 95% B to 30% B over 6 min, 30% B for 4 min (total time: 30 min). Flow: 1 mL/min. Solvent A: water with 0.1% formic acid. Solvent B: acetonitrile with 0.1% formic acid.

### General procedure for trichloroacetimidamide compounds

Trichloroacetonitrile (1.5-3 mmol) was added to the corresponding 2-aminoazole (1 mmol) in a closed vial in the presence of THF (0.5 M) or ethanol (0.1 M) or neat. The mixture was stirred at 20-65°C until completion of the reaction (TLC monitoring, 6-18 h). Then, the reaction mixture was cooled down to ambient temperature and the desired product was recovered by filtration or by evaporation of the volatiles under reduced pressure. The crude product was recrystallized in methanol or ethanol or ethanol/water mixtures. Finally, the solid was washed with pentane to obtain a pure product.

### 2,2,2-trichloro-N-(6-nitrobenzo[d]thiazol-2-yl)acetimidamide (MTF231)

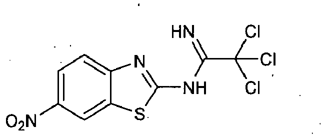

Synthesized following the general procedure using trichloroacetonitrile (0.3 mL, 3 mmol) and 2-amino-6-nitrobenzothiazole (325 mg, 1.53 mmol) in ethanol. Purified by recrystallization in ethanol resulting in an yellow solid (460 mg, 89%). ¹H NMR (200 MHz, DMSO-*d₆*): δ 9.56 (br. s, 2H, 2NH), 9.01 (s, 1H, CH-Ar), 8.30 (d, *J* = 8.3 Hz, 1H, CH-Ar), 8.01 (d, *J* = 9.1 Hz, 1H, CH-Ar). ¹³C NMR (50 MHz, DMSO-*d₆*): δ 175.85, 159.00, 155.08, 143.46, 132.74, 121.74, 121.35, 118.75, 93.71. HRMS-ESI *(m*/*z)*: [M+H]⁺ calc. for C₉H₆Cl₃N₄O₂S ⁺, 338.9271 Found: 338.9276. . HPLC purity (λ²⁵⁴): 99.3 %; *t*_{R}: 12.37 min (method 2).

### 2,2,2-trichloro-N-(4-chlorobenzo[d]thiazol-2-yl)acetimidamide (MTF235)

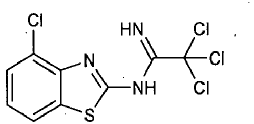

Synthesized following the general procedure using trichloroacetonitrile (0.3 mL, 3 mmol) and 2-amino-4-chlorobenzothiazole (303 mg, 1.66 mmol) in ethanol. Purified by recrystallization in methanol resulting in a yellow solid (286 mg, 88%). ¹H NMR (200 MHz, DMSO-*d₆*): δ 10.02 (br. s, 1H, NH), 9.81 (br. s, 1H, NH), 7.95 (d, *J* = 7.9 Hz, 1H, CH-Ar), 7.56 (d, *J* = 7.7 Hz, 1H, CH-Ar), 7.35 (t, *J* = 7.9 Hz, 1H, CH-Ar). ¹³C NMR (50 MHz, DMSO-*d₆*): δ 171.80, 158.57, 146.94, 133.26, 126.15, 125.14, 124.71, 120.69, 93.71. HRMS-ESI (*m*/*z*): [M+H]⁺ calc. for C₉H₅Cl₄N₃S ⁺, 327.9031; Found: 327.9027. HPLC purity (λ²⁵⁴): > 99.9%; *t*_{R}: 13.38 min, (method 2).

### 2,2,2-trichloro-N-(6-methoxybenzo[d]thiazol-2-yl)acetimidamide (MTF339)

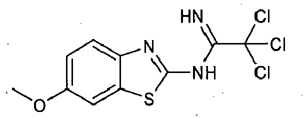

Synthesized following the general procedure using trichloroacetonitrile (0.3 mL, 3 mmol) and 2-amino-6-methoxybenzothiazole (30 mg, 1.66 mmol) in ethanol. Purified by recrystallization in methanol resulting in a brown solid (442 mg, 82%). ¹H NMR (200 MHz, DMSO-*d₆*): δ 9.66 (br. s, 2H, 2NH), 7.79 (d, *J* = 8.9 Hz, 1H, CH-Ar), 7.58 (d, *J* = 2.6 Hz, 1H, CH-Ar), 7.07 (dd, *J* = 8.9, 2.6 Hz, 1H, CH-Ar), 3.82 (s, 3H, CH₃). ¹³C NMR (50 MHz, DMSO-*d₆*): δ 168.71, 157.17, 156.78, 144.53, 133.46, 122.11, 115.22, 104.79, 93.98, 55.63. HRMS-ESI (*m*/*z*): [M+H]⁺ calc. for C₁₀H₈Cl₃N₃OS ⁺, 323.9526; Found: 323.9533. HPLC purity (λ²⁸⁰): 95.0%; *t*_{R}: 10.97 min (method 1).

### 2,2,2-trichloro-N-(6-ethoxybenzo[d]thiazol-2-yl)acetimidamide (MTF340)

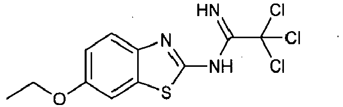

Synthesized following the general procedure using trichloroacetonitrile (0.3 mL, 3 mmol) and 2-amino-6-ethoxybenzothiazole (323 mg, 1.54 mmol) in ethanol. Purified by recrystallization in methanol resulting in a white solid (473 mg, 91%). ¹H NMR (200 MHz, DMSO-*d₆*) δ 9.68 (br. s, 2H, 2NH), 7.77 (d, *J* = 8.9 Hz, 1H, CH-Ar), 7.55 (d, *J* = 2.5 Hz, 1H, CH-Ar), 7.05 (dd, *J* = 8.9, 2.6 Hz, 1H, CH-Ar), 4.07 (q, *J* = 6.9 Hz, 2H, CH₂), 1.35 (t, *J* = 7.0 Hz, 3H, CH₃). ¹³C NMR (50 MHz, DMSO-*d₆*) δ 168.65, 157.15, 156.03, 144.44, 133.45, 122.12, 115.58, 105.38, 93.96, 63.62, 14.65. HRMS-ESI (*m*/*z*): [M+H]⁺ calc. for C₁₁H₁₀Cl₃N₃OS⁺, 337.9682; Found: 337.9685. HPLC purity (λ²⁸⁰): > 99.9%; *t*_{R}: 12.01 min (method 1).

### N-(benzo[d]thiazol-2-yl)-2,2,2-trichloroacetimidamide (MTF341)

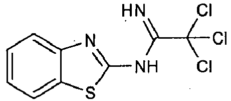

Synthesized following the general procedure overnight using trichloroacetonitrile (0.3 mL, 3 mmol) and 2-amino-benzothiazole (300 mg, 2 mmol) in ethanol. Purified by recrystallization in methanol resulting in a white solid (435 mg, 74%). ¹H NMR (200 MHz, DMSO-*d₆*): δ 9.97 (br. s, 1H, NH), 9.57 (br. s, 1H, NH), 7.98 (dd, *J* = 7.8, 0.9 Hz, 1H, CH-Ar), 7.89 (dd, *J* = 8.0, 0.7 Hz, 1H, CH-Ar), 7.48 (td, *J* = 7.7, 1.4 Hz, 1H, CH-Ar), 7.36 (td, *J* = 7.6, 1.3 Hz, 1H, CH-Ar). ¹³C NMR (50 MHz, DMSO-*d₆*): δ 170.94, 157.93, 150.41, 132.10, 126.29, 124.49, 121.72, 121.41, 93.94. HRMS-ESI *(m*/*z):* [M+H]⁺ calc. for C₉H₇Cl₃N₃S ⁺, 293.9426; Found: 293.9422. HPLC purity (λ²⁸⁰): 97.9%; *t*_{R}: 11.35 min (method 1).

### 2,2,2-trichloro-N-(4-methoxybenzo[d]thiazol-2-yl)acetimidamide (MTF571)

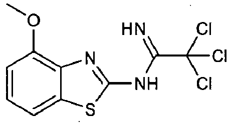

Synthesized following the general procedure using trichloroacetonitrile (0.3 mL, 3 mmol) and 2-amino-4-methoxybenzothiazole (303 mg, 1.66 mmol) in ethanol. Purified by recrystallization in pentane/diethyl ether mixtures resulting in a brown solid (443 mg, 82%). ¹H NMR (400 MHz, Chloroform-d): δ 10.58 (s, 1H, NH), 7.35 (d, *J* = 7.4 Hz, 1H, CH-Ar), 7.30 - 7.18 (m, 1H, CH-Ar), 6.87 (d, *J* = 7.6 Hz, 1H, CH-Ar), 6.85 (d, *J* = 7.6 Hz, 1H, NH), 3.97 (s, 3H, CH₃). ¹³C NMR (101 MHz, Chloroform-d): δ 170.41, 157.34, 152.46, 140.56, 134.81, 125.46, 113.70, 107.03, 94.27, 56.02. HRMS-ESI (m/z): [M+H]⁺ calc. for C₁₀H₉Cl₃N₃OS⁺ 323.9526; Found: 323.9514. HPLC purity (λ²⁵⁴): 97.7%; *t*_{R}: 7.55 min (method 2).

### 2,2,2-trichloro-N-(4-methylbenzo[d]thiazol-2-yl)acetimidamide (MTF572)

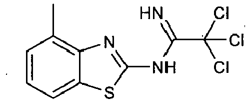

Synthesized following the general procedure using trichloroacetonitrile (0.3 mL, 3 mmol) and 2-amino-4-methylbenzothiazole (272 mg, 1.66 mmol) in ethanol. Purified by recrystallization in ethanol/water mixtures resulting in a white solid (417 mg, 82%). ¹H NMR (400 MHz, Chloroform-d): δ 10.75 (s, 1H, NH), 7.69 (dd, *J* = 6.7, 2.5 Hz, 1H, CH-Ar), 7.33 - 7.27 (m, 2H, CH-Ar), 6.88 (s, 1H, NH), 2.70 (s, 3H, CH₃). ¹³C NMR (101 MHz, Chloroform-d): δ 170.68, 157.45, 149.86, 133.06, 131.38, 126.81, 124.62, 119.01, 94.30, 18.61. HRMS-ESI (m/z):

[M+H]⁺ calc. for C₁₀H₉Cl₃N₃S ⁺: 307.9577; Found: 307.9565. HPLC purity (λ²⁵⁴): > 99.9%; *t*_{R}: 8.38 min, (method 2).

### 2,2,2-trichloro-N-(4,6-difluorobenzo[d]thiazol-2-yl)acetimidamide (MTF573)

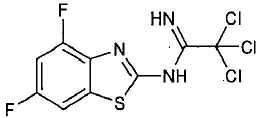

Synthesized following the general procedure using trichloroacetonitrile (0.3 mL, 3 mmol) and 2-amino-4,6-difluorobenzothiazole (186 mg, 1 mmol) in THF. Purified by washing with pentane resulting in a yellow solid (275 mg, 83%). ¹H NMR (400 MHz, Chloroform-d): δ 10.45 (s, 1H, NH), 7.29 (ddd, *J* = 7.9, 2.4, 1.2 Hz, 1H, CH-Ar), 6.95 (td, *J* = 9.8, 2.4 Hz, 1H, CH-Ar), 6.95 (br.s, 1H, NH). ¹³C NMR (101 MHz, Chloroform-d): δ 171.36, 160.89, 157.96, 155.66, 153.10, 135.97, 103.75, 102.18, 94.01. ¹⁹F NMR (376 MHz, Chloroform-d): δ -113.21 (td, *J* = 8.8, 4.9 Hz), -119.18 (dd, *J* = 10.2, 5.0 Hz). HRMS-ESI (m/z): [M+H]⁺ calc. for C₉H₅Cl₃N₃F₂S ⁺: 329.9232; Found: 329.9219. HPLC purity (λ²⁵⁴): >99.9 %; *t*_{R}: 7.93 min (method 2).

### 2,2,2-trichloro-N-(5,6-dimethylbenzo[d]thiazol-2-yl)acetimidamide (MTF574)

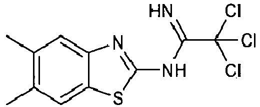

Synthesized following the general procedure using trichloroacetonitrile (0.3 mL, 3 mmol) and 2-amino-5,6-dimethylbenzothiazole (295 mg, 1.66 mmol) in ethanol. Purified by recrystallization in ethanol/water mixtures resulting in a yellow solid (477 mg, 89%). ¹H NMR (400 MHz, Chloroform-d): δ 10.56 (s, 1H, NH), 7.61 (s, 1H, CH-Ar), 7.55 (s, 1H, CH-Ar), 6.78 . (s, 1H, NH), 2.37 (s, 6H, 2 CH₃). ¹³C NMR (101 MHz, Chloroform-d): δ 170.74, 157.10, 149.34, 135.41, 134.10, 130.72, 122.14, 121.60, 94.33, 20.33, 20.23. HRMS-ESI (m/z): [M+H]⁺ calc. for C₁₁H₁₁Cl₃N₃S ⁺: 321.9722; Found: 321.9733. HPLC purity (λ²⁵⁴): 97.4%; *t*_{R}: 8.97 min, (method 2).

### 2,2,2-trichloro-N-(6-chlorobenzo[d]thiazol-2-yl)acetimidamide (MTF575)

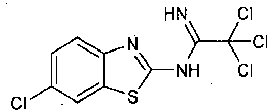

Synthesized following the general procedure using trichloroacetonitrile (0.3 mL, 3 mmol) and 2-amino-6-chlorobenzothiazole (306 mg, 1.66 mmol) in ethanol. Purified by recrystallization in ethanol/water mixtures resulting in an offwhite solid (322 mg, 59%). ¹H NMR (400 MHz, Chloroform-*d*): δ 10.46 (s, 1H, NH), 7.86 - 7.62 (m, 2H, CH-Ar), 7.39 (dd, *J* = 8.7, 2.1 Hz, 1H, CH-Ar), 6.87 (s, 1H, NH). ¹³C NMR (101 MHz, Chloroform-d): δ 171.99, 157.84, 149.26, 134.51, 130.36, 127.00, 122.51, 121.21, 94.10. HRMS-ESI (m/z): [M+H]⁺ calc. for C₉H₆Cl₄N₃S ⁺: 327.9031; Found: 327.9016. HPLC purity (λ²⁵⁴): > 99.9%; *t*_{R}: 8.56 min (method 2).

### 2,2,2-trichloro-N-(6-methylbenzo[d]thiazol-2-yl)acetimidamide (MTF576)

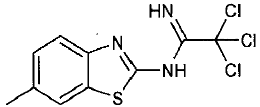

Synthesized following the general procedure using trichloroacetonitrile (0.5 mL, 5 mmol) and 2-amino-6-methylbenzothiazole (272 mg, 1.66 mmol) in ethanol. Purified by recrystallization in ethanol/water mixtures resulting in a grey solid (488 mg, 95%). ¹H NMR (400 MHz, Chloroform-d): δ 10.59 (s, 1H, NH), 7.70 (d, *J* = 8.3 Hz, 1H, CH-Ar), 7.58 (s, 1H, CH-Ar), 7.22 (s, 1H, CH-Ar), 6.82 (s, 1H, NH), 2.47 (s, 3H, CH₃). ¹³C NMR (101 MHz, Chloroform-d): δ 170.76, 157.27, 148.71, 134.76, 133.47, 127.75, 121.40, 121.35, 96.89, 21.68. HRMS-ESI (m/z): [M+H]⁺ calc. for C₁₀H₉Cl₃N₃S ⁺: 307.9577; Found: 307.9566. HPLC purity (λ²⁵⁴): 99.1%; *t*_{R}: 8.46 min (method 2).

### 2,2,2-trichloro-N-(6-hydroxybenzo[d]thiazol-2-yl)acetimidamide (MTF577)

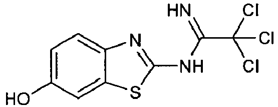

Synthesized following the general procedure using trichloroacetonitrile (1.1 mL, 11 mmol) and 2-amino-6-hydroxybenzothiazole (464 mg, 3 mmol) in ethanol. Purified by recrystallization in ethanol/water mixtures resulting in a grey solid (744 mg, 86%). ¹H NMR (400 MHz, Acetone-*d₆*): δ 10.26 (s, 1H, NH), 8.69 (s, 1H, OH), 8.43 (s, 1H, NH), 7.69 (d, *J* = 8.7 Hz, 1H, CH-Ar), 7.35 (s, 1H, CH-Ar), 7.01 (d, *J* = 8.8 Hz, 1H, CH-Ar). ¹³C NMR (101 MHz, Acetone-*d₆*): δ 169.48, 158.08, 156.06, 145.32, 135.10, 123.26, 116.46, 107.44, 96.21. HRMS-ESI (m/z): [M+H]⁺ calc. for C₉H₇Cl₃N₃OS ⁺: 309.9369; Found: 309.9356. HPLC purity (λ²⁵⁴): > 99.9%; *t*_{R}: 6.57 min (method 2).

### 2,2,2-trichloro-N-(6-ethylcarboxylatebenzo[d]thiazol-2-yl)acetimidamide (MTF578)

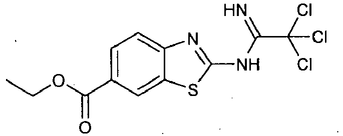

Synthesized following the general procedure using trichloroacetonitrile (6 mL, 59 mmol) and 2-amino-6-ethylcarboxylatebenzothiazole (4.43 mg, 19 mmol) in ethanol. Purified by recrystallization in ethanol resulting in an orange solid (3.046 g, 56%). ¹H NMR (400 MHz, Benzene-*d₆*): δ 10.51 (s, 1H, NH), 8.42 (d, *J* = 1.7 Hz, 1H, CH-Ar), 8.01 (dd, *J* = 8.5, 1.7 Hz, 1H, CH-Ar), 7.73 (d, *J* = 8.5 Hz, 1H, CH-Ar), 6.88 (s, 1H, NH), 4.31 (q, *J* = 7.1 Hz, 2H, CH₂), 1.32 (t, *J* = 7.1 Hz, 3H, CH₃). ¹³C NMR (101 MHz, Benzene-*d₆*): δ 225.40, 217.21, 209.15, 204.73, 184.00, 178.49, 177.59, 174.56, 172.17, 144.94, 112.25, 65.41. HRMS-ESI (m/z): [M+H]⁺ calc. for C₁₂H₁₁Cl₃N₃O₂S ⁺: 365.9632; Found: 365.9615. HPLC purity (λ²⁵⁴): 99.7 %; *t*_{R}: 8.46 min (method 2).

### 2,2,2-trichloro-N-(6-trifluoromethoxy-1,3-benzothiazol-2-yl)acetimidamide (MTF580)

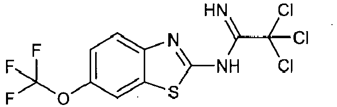

Synthesized following the general procedure using trichloroacetonitrile (0.5 mL, 5 mmol) and 2-amino-6-trifluoromethoxybenzothiazole (468 mg, 2 mmol) in THF. Purified by recrystallization in ethanol resulting in an orange solid (298 mg, 79%). ¹H NMR (400 MHz, Acetone-*d₆*): δ 10.34 (s, 1H, NH), 8.73 (s, 1H, NH), 8.02 (s, 1H, CH-Ar), 7.95 (d, *J* = 8.8 Hz, 1H, CH-Ar), 7.44 (d, *J* = 8.8 Hz, 1H, CH-Ar). ¹³C NMR (101 MHz, Acetone-*d₆*): δ 173.64, 159.24, 150.72, 146.49, 134.79, 123.48, 122.82, 120.91, 115.54, 94.86. ¹⁹F NMR (376 MHz, Acetone-*d₆*): δ -58.50. HRMS-ESI (m/z): [M+H]⁺ calc. for C₁₀H₆F₃Cl₃N₃OS ⁺: 377.9244; Found: 377.9228. HPLC purity (λ²⁵⁴): 95.4%; *t*_{R}: 8.77 min (method 2).

### 2,2,2-trichloro-N-(6-bromobenzo[d]thiazol-2-yl)acetimidamide (MTF588)

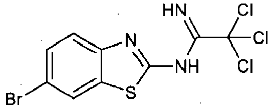

Synthesized following the general procedure using trichloroacetonitrile (0.5 mL, 5 mmol) and 2-amino-6-bromobenzothiazole (460 mg, 2 mmol) in ethanol. Purified by recrystallization in ethanol/water mixtures resulting in a beige solid (471 mg, 63%). ¹H NMR (400 MHz, Chloroform-d): δ 10.50 (s, 1H, NH), 7.93 (d, *J* = 6.2 Hz, 1H, CH-Ar), 7.67 (d, *J* = 6.6 Hz, 1H, CH-Ar), 7.61 - 7.44 (m, 1H, CH-Ar), 6.88 (s, 1H, NH). ¹³C NMR (101 MHz, Chloroform-d): δ 171.98, 157.92, 149.56, 134.94, 129.71, 124.11, 122.86, 117.91, 94.08. HRMS-ESI (m/z): [M+H]⁺ calc. for C₉H₆Cl₃BrN₃S ⁺: 371.8526; Found: 371.8511. HPLC purity(λ²⁵⁴): >99.9 %; *t*_{R}: 8.75 min (method 2).

### 2,2,2-trichloro-N-(4-bromobenzo[d]thiazol-2-yl)acetimidamide (MTF589)

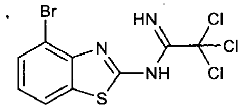

Synthesized following the general procedure using trichloroacetonitrile (0.5 mL, 5 mmol) and 2-amino-4-bromobenzothiazole (458 mg, 2 mmol) in ethanol. Purified by recrystallization in ethanol/water mixtures resulting in a white solid (508 mg, 68%). ¹H NMR (400 MHz, Chloroform-d): δ 10.77 (s, 1H, NH), 7.71 (d, *J* = 7.1 Hz, 1H, CH-Ar), 7.62 (d, *J* = 7.0 Hz, 1H, CH-Ar), 7.25 - 7.12 (m, 1H, CH-Ar), 7.01 (s, 1H, NH). ¹³C NMR (101 MHz, Chloroform-d): δ 171.86, 158.28, 148.84, 133.85, 129.43, 125.45, 120.70, 115.16, 94.13. HRMS-ESI (m/z): [M+H]⁺ calc. for C₉H₆Cl₃BrN₃S ⁺: 371.8526; Found: 371.8511. HPLC purity (λ²⁵⁴): 98.7 %; t_{R}: 8.57 min (method 2).

### 2,2-dichloro-N-(4-methylbenzo[d]thiazol-2-yl)acetimidamide (MTF619)

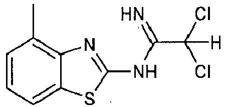

2-amino-4-methylbenzothiazole (164 mg; 1 mmol) was added slowly to dichloroacetonitrile (0.5 mL, 5 mmol) and the reaction mixture was heated at 50°C overnight. After 18 h (TLC monitoring), the crude reaction mixture was concentrated under reduced pressure to remove the excess of dichloroacetonitrile and to afford the pure product as pale yellow solid (265 mg, 97%). ¹H NMR (400 MHz, Chloroform-d): δ 10.40 (s, 1H, NH), 7.63 (d, *J* = 6.7 Hz, 1H, CH-Ar), 7.24 (d, J = 7.2 Hz, 2H, CH, CH-Ar), 6.69 (s, 1H, NH), 6.27 (s, 1H, CHCl₂), 2.66 (s, 3H, CH₃). ¹³C NMR (101 MHz, Chloroform-d): δ 170.70, 158.28, 150.08, 132.80, 131.26, 126.77, 124.49, 118.92, 69.49, 18.58. HRMS-ESI (m/z): [M+H]⁺ calc. for C₁₀H₁₀Cl₂N₃S ⁺: 273.9967; Found: 273.9788. HPLC purity (λ²⁵⁴): 97.3%; *t*_{R}: 11.96 min (method 1).

### 2,2-dibromo-N-(4-methylbenzo[d]thiazol-2-yl)acetimidamide (MTF620)

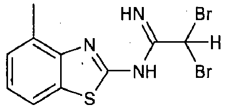

2-amino-4-methylbenzothiazole (164 mg, 1 mmol) was added slowly to dibromoacetonitrile (0.4 mL, 5 mmol) and the reaction mixture was heated at 50°C overnight. After 18 h (TLC monitoring), the crude reaction mixture was concentrated under reduced pressure to remove the excess of dibromoacetonitrile and the resulting mixture was purified by flash chromatography (cyclohexane/Ethyl Acetate, 95/5, v/v) to afford the pure product as orange solid (304 mg, 84%). ¹H NMR (400 MHz, Chloroform-d): δ 10.41 (s, 1H, NH), 7.60 (dd, J= 7.1, 1.6 Hz, 1H, CH, CH-Ar), 7.21 (d, *J* = 7.0 Hz, 2H, CH, CH-Ar), 6.69 (s, 1H, NH), 6.15 (s, 1H, CHBr₂), 2.64 (s, 3H, CH₃). ¹³C NMR (101 MHz, Chloroform-d): δ 170.43, 159.00, 150.08, 132.81, 131.23, 126.75, 124.45, 118.91, 38.69, 18.59. HRMS-ESI (m/z): [M+H]⁺ calc. for C₁₀H₁₀Br₂N₃S ⁺: 361.8957; Found: 361.8940. HPLC purity (λ²⁵⁴): 95.1%; *t*_{R}: 12.25 min (method 2).

### N-(4-methylbenzo[d]thiazol-2-yl)benzimidamide (MTF621)

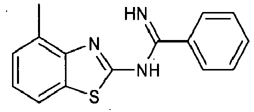

In a closed tube, 2-amino-4-methylbenzothiazole (164 mg, 1 mmol) and aluminium chloride (133 mg, 1 mmol) were mixed and benzonitrile (2 mL, 20 mmol) was added slowly. The reaction mixture was heated at 100°C overnight. After 18 h (TLC monitoring), the product was precipitated by adding diethyl ether, recovered by filtration, and purified by recrystallisation in ethanol to afford the pure product as white solid (188 mg, 70%)._¹H NMR (400 MHz, DMSO-*d*₆): δ 11.25 (s, 1H, NH), 11.10 (s, 1H, NH), 8.12 - 8.05 (m, 2H, CH-Ar), 7.90 (dd, *J* = 7.2, 2.0 Hz, 1H, CH-Ar), 7.77 (d, *J* = 7.3 Hz, 1H, CH-Ar), 7.66 (t, *J* = 7.7 Hz, 2H, CH-Ar), 7.38 - 7.29 (m, 2H, CH-Ar), 2.67 (s, 3H, CH₃). ¹³C NMR (101 MHz, DMSO-*d*₆): δ 162.10, 147.96, 133.81, 131.01, 130.84, 129.09 (2C), 128.80 (2C), 127.25 (2C), 125.21, 119.50 (2C), 18.00. HRMS-ESI (m/z): [M+H]⁺ calc. for C₁₅H₁₄N₃S ⁺: 268.0903; Found: 268.0892. HPLC purity (λ²⁵⁴): >99.9 %; *t*_{R}: 7.62 min (method 1).

### N-(4-methylbenzo[d]thiazol-2-yl)thiophene-2-carboximidamide (MTF623)

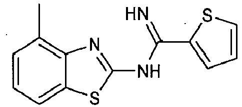

In a closed tube, 2-amino-4-methylbenzothiazole (164 mg, 1 mmol) and aluminum chloride (133 mg, 1 mmol) were mixed and 2-thiophenecarbonitrile (1.9 mL, 20 mmol) was added slowly. Then the reaction mixture was heated at 100°C overnight. After 18 h (TLC monitoring), the product was precipitated by adding diethyl ether, recovered by filtration, and purified by recrystallisation in ethanol afford the pure product as green solid (186 mg, 68%). ¹H NMR (400 MHz, DMSO-*d*₆): δ 10.40 (s, 1H, NH), 10.02 (s, 1H, NH), 8.22 (m, 1H, CH-Thio), 7.99 (m, 1H, CH-Thio), 7.77 (dd, *J* = 7.8, 1.5 Hz, 1H, CH-Thio), 7.31 - 7.21 (m, 3H, CH-Ar), 2.60 (s, 3H, CH₃). ¹³C NMR (101 MHz, DMSO-*d*₆): δ 155.05,148.22, 133.14, 131.24, 130.26, 129.23, 128.04 (2C), 126.35, 123.66, 118.63 (2C), 17.55. HRMS-ESI(m/z): [M+H]⁺ calc. for C₁₃H₁₂N₃S₂⁺: 274.0394; Found: 274.0461. HPLC purity (λ²⁵⁴): 96.7%; *t*_{R}: 11.22 min (method 1).

### N-(4-methylbenzo[d]thiazol-2-yl)isobutyrimidamide (MTF625)

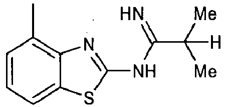

In a closed tube, 2-amino-4-methylbenzothiazole (164 mg, 1 mmol) and aluminum chloride (133 mg, 1 mmol) were mixed and isobutyronitrile (1.8 mL, 20 mmol) was added slowly. Then the reaction mixture was stirred at 100°C. After 48 h (TLC monitoring), the product was precipitated by adding diethyl ether, recovered by filtration, and purified by recrystallisation in ethanol to afford the pure product as pale-yellow solid (148 mg, 63%). ¹H NMR (400 MHz, DMSO-*d*₆): δ 10.31 (s, 1H, NH), 9.82 (s, 1H, NH), 7.86 - 7.69 (m, 1H, CH-Ar), 7.34 - 7.15 (m, 2H, CH-Ar), 2.88 (s, 1H, CH), 2.58 (s, 3H, CH₃), 1.29 (d, *J* = 6.9 Hz, 6H, 2 CH₃). ¹³C NMR (101 MHz, DMSO-*d*₆): δ 171.49, 149.05, 130.81, 130.15, 126.78, 124.26, 119.13 (2C), 34.05, 19.79 (2C), 18.04. HRMS-ESI (m/z): [M+H]⁺ calc. for C₁₂H₁₅N₃S ⁺: 234.1059; Found: 234.1052. HPLC purity (λ²⁵⁴): 95.9%; *t*_{R}: 6.17 min (method 1).

### N-(4-methylbenzo[d]thiazol-2-yl) trichloroacetamide (MTF637)

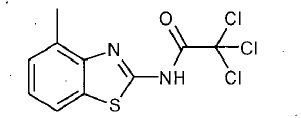

*N*-methylmorpholine (0.67 mL, 6.08 mmol) and trichloroacetyl chloride (0.68 mL, 6.08 mmol) were added to a solution 2-amino-4-methylbenzothiazole (500 mg, 3.04 mmol) in THF (7 mL) and the reaction mixture was stirred at room temperature. After 2 h (TLC monitoring), the reaction mixture was concentrated under reduced pressure and the crude mixture was purified by flash column chromatography with dichloromethane as eluent to obtain the titled product as brown powder (578.2 mg; 61%). ¹H NMR (400 MHz, Acetone-*d*₆): δ 11.16 (s, 1H, NH), 7.77 (d, *J* = 6.4 Hz, 1H, CH-Ar), 7.43 - 7.20 (m, 2H, CH-Ar), 2.63 (s, 3H, CH₃). ¹³C NMR (101 MHz, Acetone-*d*₆): δ 168.75. 168.50, 163.07, 139.19, 129.19, 127.02, 125.53, 120.90, 95.62, 17.85. HRMS-ESI (m/z): [M+H]⁺ calc. for C₁₀H₈N₂Cl₃OS ⁺: 308.94174; Found: 308.94056. HPLC purity (λ²⁵⁴): 96.4 %; *t*_{R}: 11.13 min (method 2).

### N-(4,6-difluorobenzo[d]thiazol-2-yl)trichloroacetamide (MTF639)

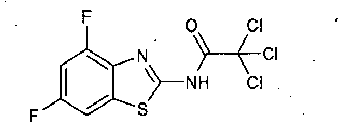

2-amino-4,6-difluoro-benzothiazole (186 mg, 1 mmol) was dissolved in THF (2 mL) and trichloroacetylchloride (0.56 mL, 5 mmol) was added slowly at ambient temperature. The reaction mixture was stirred at 40°C for 4 h. The crude reaction mixture was evaporated under reduced pressure to remove the solvent and the excess of trichloroacetylchloride. The resulting solid was recrystallized in ethanol to afford the pure product as white solid (303 mg, 92%). ¹H NMR (400 MHz, Chloroform-d): δ 8.42 (s, 1H, NH), 7.39 (d, *J* = 6.7 Hz, 1H, CH-Ar), 7.02 (t, *J* = 9.2 Hz, 1H, CH-Ar). ¹³C NMR (101 MHz, Chloroform-d): δ 160.23, 160.11, 156.66, 154.60, 135.28, 133.9, 103.97, 102.96, 90.96. ¹⁹F NMR (376 MHz, Chloroform-d): δ -111.94, -120.08. HRMS-ESI(m/z): [M+H]⁺ calc. for C₉H₄Cl₃N₂OSF₂⁺: 330.9000; Found: 330.9066. HPLC purity (λ²⁵⁴): 95.4%; *t*_{R}: 11.53 min (method 2).

### N-(4,6-dichlorobenzo[d]thiazol-2-yl)trichloroacetamide (MTF640)

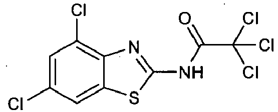

2-amino-4,6-dichlorobenzothiazole (218 mg, 1 mmol) was dissolved in THF (2 mL) and trichloroacetylchloride (0.56 mL, 5 mmol) was added slowly at room temperature. The reaction mixture was stirred at 40°C for 5 h. The crude reaction mixture was evaporated under reduced pressure to remove the excess of trichloroacetylchloride, followed by precipitation of the product the addition of pentane. The resulting solid was recrystallized in ethanol to afford the pure product as beige solid (316 mg, 88%).¹H NMR (400 MHz, DMSO-*d*₆) δ 8.30 (s, 1H, NH), 8.14 (d, *J* = 2.5 Hz, 1H, CH-Ar), 7.66 (d, *J* = 2.0 Hz, 1H, CH-Ar). ¹³C NMR (101 MHz, DMSO-*d*₆) δ 163.31, 159.05, 141.98, 134.30, 133.43, 128.48, 126.70, 121.10, 92.17. LCMS-ESI (m/z): [M+H]⁺ calc. for C₃H₃Cl₃N₂OS ⁺: 362.8; Found: 363.3. HPLC purity (λ²⁵⁴): 98.2%; *t*_{R}: 12.99 min (method 1).

### N-(4-flurobenzo[d]thiazol-2-yl)trichloroacetamide (MTF641)

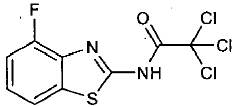

2-amino-4-fluorobenzothiazole (168 mg, 1 mmol) was dissolved in THF (2 mL) and trichloroacetylchloride (0.56 mL, 5 mmol) was added slowly at room temperature. The reaction mixture was stirred at 40°C for 2 h. The crude reaction mixture was evaporated under reduced pressure to remove the solvent and the excess of trichloroacetylchloride. The solid was recrystallized in ethanol to afford the pure product as brown solid (304 mg, 97%). ¹H NMR (400 MHz, chloroform-d): δ 8.68 (s, 1H, NH), 8.40 (t, *J* = 8.2 Hz, 1H, CH-Ar), 7.49 - 7.34 (m, 2H, CH-Ar). ¹³C NMR (101 MHz, chloroform-d): δ 159.40, 152.98, 127.12, 126.42, 122.66, 121.59, 117.34, 109.61, 92.17. ¹⁹F NMR (376 MHz, Chloroform-d): -126.10. HRMS-ESI(m/z): [M+H]⁺ calc. for C₉H₅Cl₃N₂OSF⁺: 310.9094; Found: 310.9020. HPLC purity (λ²⁵⁴): >99.9 %; *t*_{R}: 10.61 min (method 2).

### 2,2,2-trichloro-N-(4,6-dichlorobenzo[d]thiazol-2-yl)acetimidamide (MTF642)

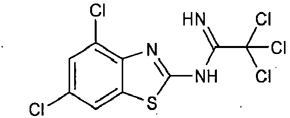

Synthesized following the general procedure using trichloroacetonitrile (0.3 mL, 3 mmol) and 2-amino-4,6-dichlorobenzothiazole (219 mg, 1 mmol) in THF. Purified by trituration in pentane resulting in a yellow solid (344 mg, 95%). ¹H NMR (400 MHz, Chloroform-d): δ 10.63 (s, 1H, NH), 7.67 (d, *J* = 1.9 Hz, 1H, CH-Ar), 7.46 (d, *J* = 2.0 Hz, 1H, CH-Ar), 7.02 (s, 1H, NH). ¹³C NMR (101 MHz, Chloroform-d): δ 172.42, 158.50, 146.42, 135.11, 130.23, 126.82, 126.74, 119.82, 93.99. HRMS-ESI(m/z): [M+H]⁻ calc. for C₉H₄Cl₅N₃S ⁻: 361.8641; Found: 361.8464. HPLC purity (λ²⁵⁴): > 99.9%; *t*_{R}: 14.60 min (method 1).

### 2,2-dibromo-N-(4-bromobenzo[d]thiazol-2-yl)acetimidamide (MTF643)

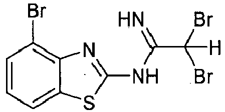

4-bromo-1,3-benzothiazol-2 amine (1.0 mmol, 229 mg, 1 eq) was dissolved in THF (2 mL), dibromoacetonitrile (5.0 mmol, 0.45 mL, 5 eq) was added slowly at ambient temperature and the reaction mixture was heated at 60°C. After 18 h (TLC monitoring), the crude reaction mixture was concentrated under reduced pressure to remove the excess of dibromoacetonitrile and the resulting mixture was purified by flash chromatography (cyclohexane/Ethyl Acetate, 95/5, v/v) to afford the pure product as yellow solid (269 mg, 63%). ¹H NMR (400 MHz, Chloroform-d): δ 10.49 (s, 1H, NH), 7.69 (dd, J = 8.0, 1.1 Hz, 1H, CH-Ar), 7.61 (dd, J = 7.9, 1.0 Hz, 1H, CH-Ar), 7.16 (t, J = 7.9 Hz, 1H, CH-Ar), 6.76 (s, 1H, NH), 6.10 (d, J = 1.0 Hz, 1H, CH-Br2). ¹³C NMR (101 MHz, Chloroform-d) δ 171.81, 159.87, 149.18, 133.74, 129.43, 125.32, 120.66, 115.06, 38.49. HRMS-ESI(m/z): [M+H]+ calc. for C₉H₇Br₃N₃S⁺: 427.7885; Found: 427.7867.

### 2,2,2-trichloro-N-(4-fluorobenzo[d]thiazol-2-yl)acetimidamid (MTF645)

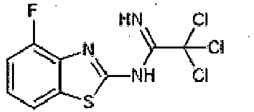

Synthesized following the general procedure using trichloroacetonitrile (0.3 mL, 3 mmol) and 2-amino-4-fluorobenzothiazole (168 mg, 1 mmol) in THF. Purified by silica gel flash chromatography (cyclohexane/EtOAc, 10/9 to 9/1, v/v) resulting in a yellow solid (237 mg, 76%). ¹H NMR (400 MHz, Chloroform-d): δ 10.56 (s, 1H, NH), 7.52 (dd, *J* = 8.0, 1.0 Hz, 1H, CH-Ar), 7.23 (td, *J* = 8.1, 4.7 Hz, 1H, CH-Ar), 7.15 - 7.07 (m, 1H, CH-Ar), 6.90 (s, 1H, NH). ¹³C NMR (101 MHz, Chloroform-d): δ 171.91, 158.05, 154.84, 139.31, 135.74, 118.14, 117.21, 94.09, 27.03. ¹⁹F NMR (Chloroform-d): -123.35. HRMS-ESI (m/z): [M+H]⁻ calc. for C₉H₅Cl₃FN₃S⁻: 309.9170; Found: 309.9180. HPLC purity (λ²⁵⁴): > 99.9%; *t*_{R}: 12.38 min (method 1).

### 2,2,2-trichloro-N-(4,6-dimethylbenzo[d]thiazol-2-yl)acetimidamide (MTF646)

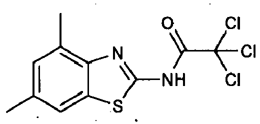

2,3-dihydro-4,6-dimethyl-2-iminobenzothiazole (178 mg, 1 mmol) was dissolved in THF (2 mL) and trichloroacetylchloride (0.56 mL, 5 mmol) was added slowly at room temperature. Then the reaction mixture was stirred at 40°C for 2 h. The crude reaction mixture was evaporated under reduced pressure to remove the excess of trichloroacetylchloride. The solid was recrystallized in ethanol to afford the pure product as beige solid (276 mg, 85%). ¹H NMR (400 MHz, Acetone-*d*₆): δ 7.64 (s, 1H, CH-Ar), 7.24 (s, 1H, CH-Ar), 2.61 (s, 3H, CH₃), 2.44 (s, 3H, CH₃). ¹³C NMR (101 MHz, Acetone-*d*₆): δ 167.83, 167.20, 136.41, 136.14, 130.85, 129.00, 126.64, 120.71, 94.82, 21.28, 17.66. HRMS-ESI (m/z): [M+H]⁺ calc. for C₁₁H₁₀Cl₃N₂OS ⁺: 322.9501; Found: 322.9568. HPLC purity (λ²⁵⁴): 95.1%; *t*_{R}: 11.95 min (method 1).

### 2,2,2-trichloro-N-(4-chlorobenzo[d]thiazol-2-yl)acetamide (MTF647)

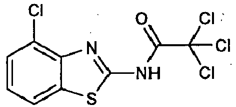

2-amino-4-chlorobenzothiazole (184 mg, 1 mmol) was dissolved in THF (2 mL) and trichloroacetylchloride (0.56 mL, 5 mmol) was added slowly at room temperature. The reaction mixture was stirred at 40°C for 2 h. The crude reaction mixture was evaporated under reduced pressure to remove the solvent and the excess of trichloroacetylchloride. The resulting solid was recrystallized in ethanol to afford the pure product as white solid (315 mg, 96%). ¹H NMR (400 MHz, Acetone-*d*₆): δ 8.01 (d, *J* = 7.9 Hz, 1H, CH-Ar), 7.59 (d, *J* = 7.9 Hz, 1H, CH-Ar), 7.42 (t, J= 7.9 Hz, 1H, CH-Ar). ¹³C NMR (101 MHz, Acetone-*d*₆): δ 164.16, 162.98, 143.15, 133.30, 127.91, 126.34, 124.74, 121.88, 93.18. HRMS-ESI (m/z): [M+H]⁺ calc. for C₉H₅Cl₄N₂OS⁺: 328.8871; Found: 328.8865. HPLC purity (λ²⁵⁴): 95.58%; *t*_{R}: 11.40 min (method 1).

### 2,2,2-trichloro-N-(4,6-dimethylbenzo[d]thiazol-2-yl)acetimidamide (MTF648)

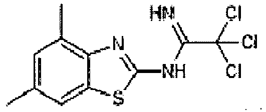

Synthesized following the general procedure using trichloroacetonitrile (0.3 mL, 3 mmol) and 2,3 dihydro-4,6 dimethyl-2-iminobenzothiazole (178 mg, 1 mmol) in THF. Purified by silica gel flash chromatography (cyclohexane/ethyl acetate, 100/0 to 95/5, v/v) resulting in an orange solid (144 mg, 44%). ¹H NMR (400 MHz, Chloroform-d): δ 10.65 (s, 1H, NH), 7.43 (s, 1H, CH-Ar), 7.07 (s, 1H, CH-Ar), 6.78 (s, 1H, NH), 2.61 (s, 3H, CH₃), 2.43 (s, 3H, CH₃). ¹³C NMR (101 MHz, Chloroform-d): δ 169.77, 157.11, 147.89, 134.66, 133.21, 130.89, 128.43, 118.81, 94.35, 21.65, 18.51. HRMS-ESI(m/z): [M+H]⁻ calc. for C₁₁H₁₁Cl₃N₃S ⁻: 320.9656; Found: 320.9427. HPLC purity (λ²⁵⁴): > 99.9%; *t*_{R}: 13.65 min (method 1).

### Example 2 : antibacterial activity

The inhibition of the growth of two Staphylococcus aureus strains (RN4240, modified laboratory strain, non-pathogenic; and S25, pathogenic strain obtained from patients) by the presence of our compounds were used to evaluate their efficacy as bacteriostatic and/or bactericide compounds.

All compounds presented were dissolved in DMSO (10 mM stock solution) and tested initially at 50 µM concentration. The bacterial growth was measured after 5 h and 20 h incubation at 37°C with the compounds. As positive control, the antibiotics ampicillin and kanamycin were used.

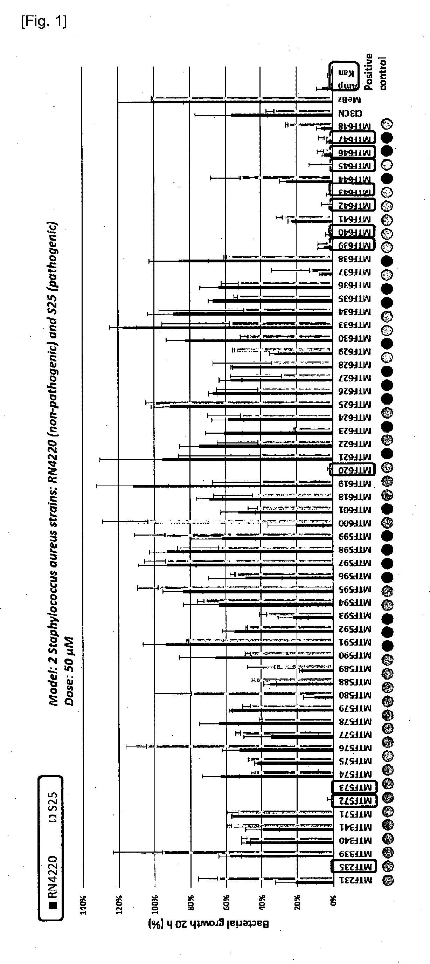

Results for 53 compounds are represented at figure 1.

Compounds substituted by 4-methyl, 4-chloro and 4,6-difluoro exhibit total growth inhibition after 20 h of both strains, and 4,6-dichloro compound shows bacterial growth < 3%. The compounds containing the 4-fluoro and 4,6-dimethyl substituted benzothiazole moiety also demonstrate good inhibition for the non-pathogenic strain RN4220.

The Minimal Inhibitory Concentration (MIC) was determined for the compounds that showed no growth for both strains at 20 h (MTF235, MTF572 and MTF573). MIC is defined as the smallest concentration of a compound that inhibits the growth of bacteria. For that, 6 doses were tested (1, 5, 12.5, 25, 50, 100 µM) for both strains incubated at 37° C, for 20 h. Besides, small aliquots of those cultures that did not grow after 20 h at determined condition were taken and inoculated in a fresh bacterial media to evaluate the Minimal Bactericide Concentration (MBC) after 24 h.

No bacterial growth indicates that the compound is not only bacteriostatic (inhibits the growth for certain time) but it is also bactericide (promotes the death of the bacteria)Results of bacteriostatic vs bactericide activity are summarized in table 1 below

| | Bacteriostatic, 20h | | Bactericide, 24h | |
|---|---|---|---|---|
| compound | MIC RN4220 (µM) | MIC S25 (µM) | MBC RN4220 (µM) | MBC S25 (µM) |
| MTF235 | 25 | 25 | 50 | 50 |
| MTF572 | 40 | 40 | 100 | - |
| MTF573 | 40 | 50 | 50 | 50 |
| MTF642 | 12,5 | 50 | 12,5 | 50 |
| MTF643 | 25 | 25 | 25 | 50 |
| MTF640 | 12,5 | 25 | 50 | 50 |

Minimum inhibitory concentration (MIC, incubation for 20 h) and maximum bactericide concentration (MBC, incubation for 24 h) for S. aureus strains RN4220 and S25.

### 2 Staphylococcus aureus strains:

RN4220 (non-pathogenic)
S25 (pathogenic)
MIC: minimal inhibitory concentration
MBC: minimal bactericide concentration

### Example 3 cytotoxicity

**HEK293:** 24 h in the presence of the bactericide compounds or DMSO
**Method of evaluation:** CyQUANT^{™} LDH Cytotoxicity Assay Kit.

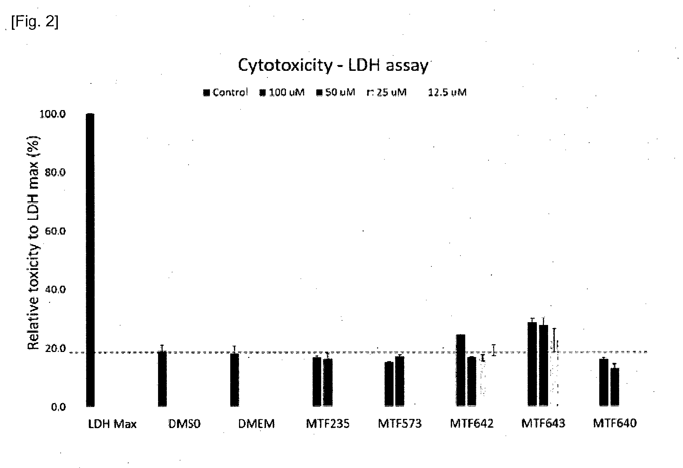

Results are shown on figure 2.

Cytotoxicity through extracellular lactate dehydrogenase (LDH) quantification upon incubation of the compounds with HEK 293T cells at the same range of concentration as MIC and MBC. LDH: positive control, DMEM: negative control. N = 3.

Preliminary safety of these compounds towards host cells was evaluated by using a extracellular lactate dehydrogenase (LDH) release model with human embryonic kidney 293 cells (HEK293). Compounds **MTF235, MTF573, MTF640, MTF642** and **MTF643** displayed no cytotoxicity activity with similar extracellular lactate dehydrogenase (LDH) quantification levels than the DMSO control upon incubation at their active doses.

## Claims

1. Compound with the following formula I: 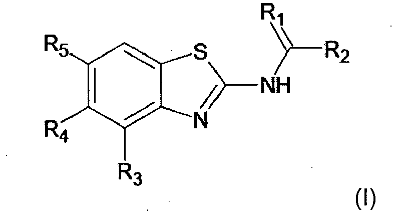 wherein
- R1 represents O or NH,
- R2 represents a linear, branched or cyclic alkyl comprising from 1 to 8 carbon atoms, possibly interrupted by a group selected from -O-, -S-, -(C=O)-, NH and possibly being substituted by one or more halogens;
an alkylene comprising from 1 to 8 carbon atoms and possibly being substituted by one or more halogens,
a phenyl or an arylene,
- R3, R4 and R5 represent independently -H, a halogen, a linear or branched alkyl comprising from 1 to 8 carbon atoms, possibly being substituted by one or more halogens, CN, -NO₂, OX₁ with X₁ being selected from -H, linear or branched alkyl comprising from 1 to 8 carbon atoms, possibly being substituted by one or more halogens, COOR6, OCOR6, SO2NR6R7, NR6R7, NR6COR7, or OR6, with R6 and R7 being identical or different and R6 and R7 representing H or an alkyl comprising from 1 to 8 carbon atoms,
for use as a medicament.

2. Compound with the following formula I: 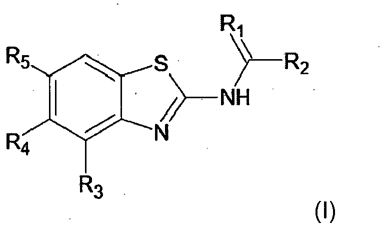 wherein
- R1 represents O or NH,
- R2 represents a linear, branched or cyclic alkyl comprising from 1 to 8 carbon atoms, possibly interrupted by a group selected from -O-, -S-, -(C=O)-, NH and possibly being substituted by one or more halogens;
an alkylene comprising from 1 to 8 carbon atoms and possibly being substituted by one or more halogens,
a phenyl or an arylene,
- R3, R4 and R5 represent independently -H, a halogen, a linear or branched alkyl comprising from 1 to 8 carbon atoms, possibly being substituted by one or more halogens, -NO₂, or OX₁ with X₁ being selected from -H, linear or branched alkyl comprising from 1 to 8 carbon atoms, possibly being substituted by one or more halogens,
for its use as a medicament.

3. Compound of formula (I) for its use according to any one of claims 1 or 2, wherein R2 represents a linear or branched alkyl comprising 1 to 5 carbon atoms, substituted by at least one halogen.

4. Compound of formula (I) for its use according to any one of claims 1 to 3, wherein R2 represents a linear or branched alkyl comprising 1 to 5 carbon atoms, being substituted by 3 halogens.

5. Compound of formula (I) for its use according to any one of claims 1 to 4, wherein at least one of R3 or R5 represents a linear or branched alkyl comprising from 1 to 6 carbon atoms, possibly being substituted by one or more halogens, or a halogen, and R4 represents H.

6. Compound of formula (I) for its use according to any one of claims 1 to 5, wherein R3 represents a halogen or a linear or branched alkyl comprising 1 to 3 carbon atoms.

7. Compound of formula (I) for its use according to any one of claims 1 to 6, wherein R1 represents NH.

8. Compound of formula (I) for its use according to any one of claims 1 to 7, wherein R2 represents C(R'1)₃, wherein R'1 represents a halogen..

9. Compound of formula (I) for its use according to any one of claims 1 to 8, wherein at least one from R3, R4 or R5 represents -H.

10. Compound of formula (I) for its use according to any one of claims 1 to 9, having one of the following formula: 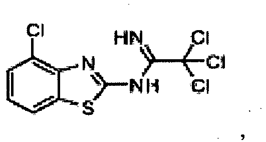 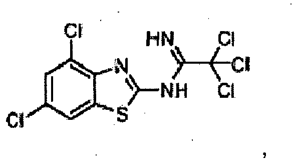 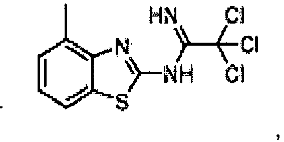 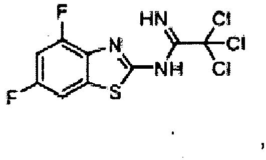 and

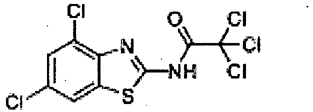

11. Compound of formula (I) for its use according to any one of claims 1 to 10, wherein the compound is used as an antibiotic.

12. Compound of formula (I) for its use according to any one of claims 1 to 10, wherein the compound is used for the treatment of infectious disease associated with at least a gram + or gram - bacteria.

13. Compound of formula (I) as defined in any one of claims 1 to 10, with the exception of compounds of the following formula

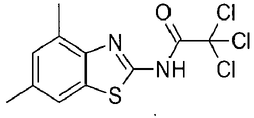

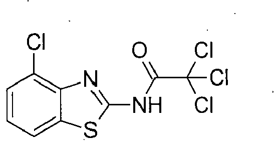

14. Compound of formula (I) 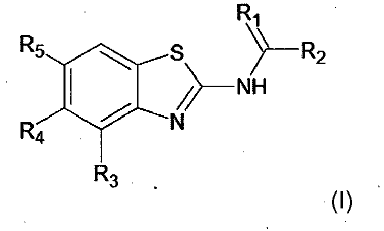 wherein R1, R2, R3, R4, R5 are as defined in any one of claims 1 to 10, said compound being complexed with at least a metallic ion.

15. Pharmaceutical composition comprising at least a compound of formula (I) 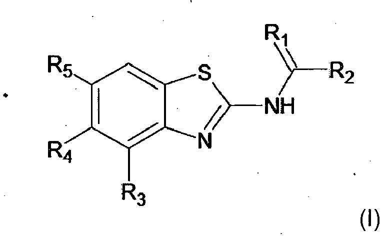 wherein R1, R2, R3, R4, R5 are as defined in any one of claims 1 to 10, a salt or a metallic complex thereof, and pharmaceutical acceptable carriers.
